# EUROPEAN PATENT SPECIFICATION

(11) **EP 1 265 556 B1**
(45) Date of publication and mention of the grant of the patent: **25.06.2008**
(21) Application number: 01916030.8
(22) Date of filing: 22.03.2001
(51) Int. Cl.: A61F 2/30

(54) **JOINT SURFACE REPLACEMENT OF THE DISTAL RADIOULNAR JOINT**
GELENKOBERFLÄCHENERSATZ DES DISTALEN RADIOULNARGELENKES
REMPLACEMENT DE SURFACE D'ARTICULATION DE L'ARTICULATION RADIO-CUBITALE DISTALE

(30) Priority: 23.03.2000 SE 0001024
(43) Date of publication of application: 18.12.2002
(73) Proprietor: Ascension Orthopedics, Inc., Austin, TX 78754-3832 (US)
(72) Inventor: KOPYLOV, Dr. Philippe, S-22465 Lund (SE); TÄGIL, Magnus, S-227 30 Lund (SE)
(74) Representative: Klusmann, Peter
(86) International application number: PCT/SE2001/000615
(87) International publication number: WO 2001/070138

(56) References cited:
- WO-A1-91/16017
- US-A- 5 951 604

## Description

### Background

The present invention relates to a joint prosthesis, below named joint surface replacement, for the distal radioulnar (DRU) joint of the forearm.

Cartilage destruction of the distal radioulnar joint is often caused by disease, such as different types of rheumatoid diseases, especially rheumatoid arthritis. Today these injuries are operated rather late in the evolution of the disease when pain evolves or mobility starts to decrease. At this time the joint most often is destroyed without remaining cartilage and with varying degrees of bone destruction. A common operation often used is the Darrach procedure, which consists of a simple resection of the ulnar head (caput ulna). The cut ulnar bone-end is now mobile and "floats" and sometimes the wrist feels unstable and painful. There is a risk for the ulna and radius to stick to each other. Sometimes the patient feels a clicking sensation, sometimes painful, when turning the forearm.

In the non-rheumatoid patients, the DRU joint most often is injured as a consequence of a distal radial fracture, causing a secondary joint surface incongruity of the distal radial ulnar joint. The incongruity may occur as a consequence of an intraarticular radial fracture extending into the DRU joint. The joint surface then heals with a step-off. Also a radial fracture, which does not extend into the DRU joint, might influence the congruity due to an angulation of the radial shaft and the radial joint surface of the DRU joint.

The consequence of an incongruity may be an osteoarthritis, which might be either symptomatic or not. Different treatment alternatives exist, none of them being particularly good. All are compromises, trading different wrist and hand functions to achieve pain relief. A common method is the Sauvee-Kapandjii procedure, where the ligaments from the ulnar tip to the radius and carpus are maintained, the ulna is resected proximally and screws keep the ulnar head to the radius. The radius together with the ulnar head now pivot, within the osteotomy defect.

Other known methods comprise the Bowers hemiresection of the ulnar end with soft tissue interposition and the Watson distal ulnar resection. Methods to resect the ulnar head and replace it with a prosthesis are also described.

The publication WO 91/16017 discloses a system for reconstructing the distal radioulnar joint (DRU-joint) in the wrist. The system includes as a substantial component a guide body anchorable to the radius and having a guide slot with a control element extending into the guide slot and adjustably secured in the ulna.

US-A-5,951,604 discloses a DRU-joint prosthesis. A brace is mounted on the radius and receives a ball which is on the end of a rod that is attached to the ulna by inserting it in the bone cavity of the ulna.

An object with the present invention has been to develop a device making it possible to use a method of operation, which would lead to better clinical results than by the different methods used today.

It is also an object of the invention to make it possible to keep the DRU joint as intact as possible.

The device, according to the invention, can be used for example for symptoms after a radial fracture, rheumatoid arthritis or other rheumatoid disease involving the distal radioulnar joint.

The present invention is defined in different aspects, in the independent claims below. The dependent claims are directed to optional and preferred features.

The motion, which is supported by the use of the prosthesis, is the pivotation (pronation/supination) of the forearm. The ulna is the non-moving and weight-bearing fundament of the distal radio-ulnar joint, while the radius is a mobile component with mostly compressing forces influencing the positioning during the pivotal movement. The radius turns around the ulnar head. Besides the distal joint surfaces between the radius and ulna, a prerequisite for the turning movement is the existence of joint surfaces also proximally in the elbow. The proximal radioulnar joint consists of the radial head and the ulna with a fossa radii and an anular ligament.

In the elbow the ulna makes a flexion or extension motion, whereas the radius rotates around it own axis. Both the radius and the ulna have a curvature that makes the middle point of the two bones to be far away from each other. Through this arrangement, the radius has enough space to be able to rotate around the ulna.

The stability depends on both the congruity of the two radio-ulnar joints as well as the ligaments of the two joints keeping the radius and ulna together. Muscle forces push the two bones together, still allowing them to make both a rolling and a translatory motion relative each other. The forces in the distal radioulnar joint thus are mainly compressive. Different parts of the ulnar head will be in contact with the joint surface of the radius as the ulnar head successively translates and rotates along the joint surface. In the two extreme positions of pronation and supination the joint surface of the radius is loaded in the volar part and the dorsal part respectively. The ligaments stop the joint from luxation.

When problems arise in the distal radioulnar joint, it is common, as previously mentioned, to simply resect the ulnar head and attach it to the radius or replace it with a prosthesis. The result of this is often that the distal position of the radius is changed since the radius is resting upon the ulna. Said change of position often makes the result of the operation less satifactory.

Through an implantation of the surface replacement according the present invention the ulna is kept intact and is thus able to support the radius without changing the position of the radius in a non favorable way.

A surface replacement designed as in the independent claim makes it possible to achieve the above mentioned objects and demands.

The invention makes it possible to preserve the ligamentous apparatus when implanting the surface replacement. This means that the compressive forces within the distal radioulnar joint are maintained also after the operation contributing to the stabilization of the joint.

### Description

Below the invention will be described more in detail by reference to the drawings below, wherein
- Fig. 1.2 the ulnar component according to the invention is shown in different perspectives,
- Fig. 3.4 the radial component according to the invention is shown in different perspectives,
- Fig. 5a, b schematic figures of the ulna and radius with the hand in its extreme rotational positions, (supination 5a and pronation 5b), is shown.
- Fig. 6a a schematic figure of the anchorage of the surface replacement according to the invention is shown,
- Fig. 6b an example of the positions of the radial and ulnar components relative each other after implantation in the distal radioulnar joint is shown,
- Fig. 7a a frontal view of an alternative design of the radial component is shown,
- Fig. 7b a side view through the section line VII-VII of fig. 7a is shown,
- Fig. 8 a perspective view of an alternative design of the ulnar component is shown,
- Fig. 8a a view of the side of the ulnar component, according to fig. 8 that faces the radial component after implantation into the distal radioulnar joint is shown,
- Fig. 8b the ulnar component, according to fig. 8 as seen in the direction of the arrow in fig. 8a is shown.

The surface replacement according to the invention comprises two parts, a radial component (1.1a) and an ulnar component (2.2a). When implanted, the two components 1 and 2 are mounted radially on the radius (6) and the ulna (7) respectively. The expression radial mount in this description means that, during the operation, the artificial components are placed at the surface of the natural joint surfaces thus forming a new surface.

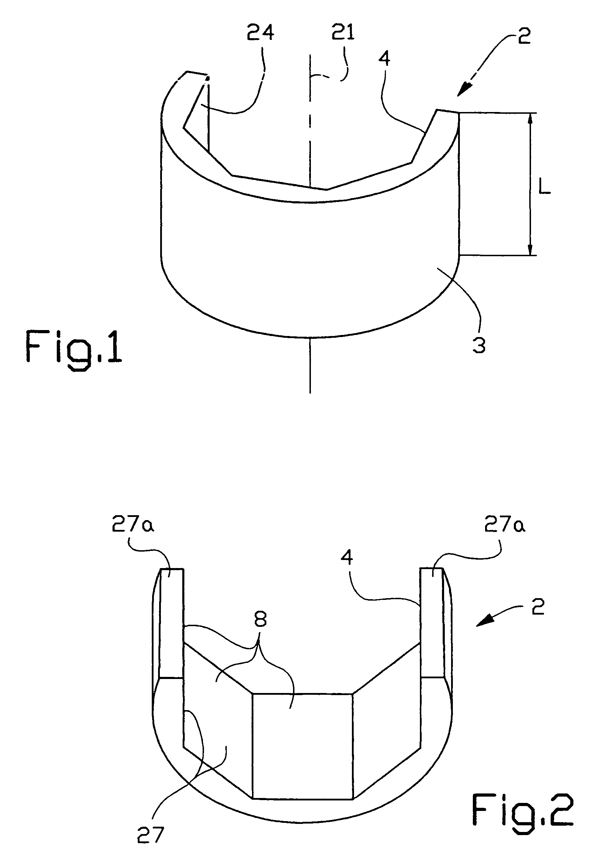

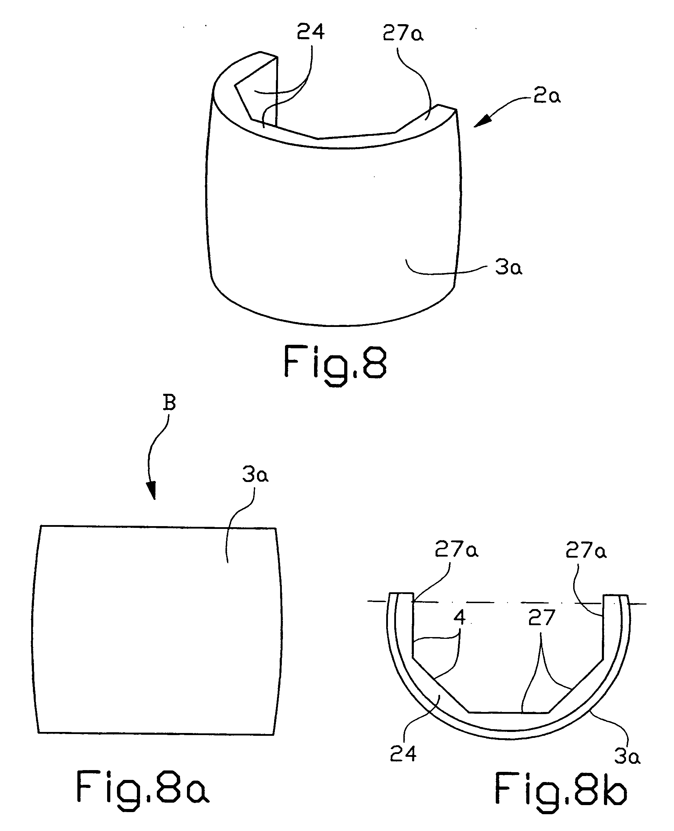

As shown in figs. 1 and 8, the ulna component (2.2a) has a body (24) with a convex outer surface (3). The outer surface (3) in fig. 1, forms a mainly semicylindrical envelope surface around a central axis (21). The direction of the central axis is below described as the direction of the axis of the ulna component. In figure 8 is shown an embodiment where the outer surface of the body (3A) is convex also in the longitudinal direction of the component. The ulnar component (2.2a) has an inner surface (4), which in the shown figures consists of rectangular fields forming angles to each other.

A transection through the body (24) through the longitudinal direction of the body shows a horseshoe-shaped appearance. The design of the outer and the inner surface implies that the body forms a number of juxtaposed and to each other integrated mainly quadratic or rectangular elements (27). The two extreme elements (27a) are normally parallel to each other and intended to point away from the radius when the component is implanted on to the ulnar head.

The ulnar component (2) has a length L (fig 1) corresponding to the length of the natural ulnar head and to the size of the normal joint surfaces distally between the radius (6) and ulna (7). The ulnar component (2.2a) is made of surgical stainless steel but can of course be made out of other materials such as pyrocarbon, ceramics, or composites. During the operation, the ulnar component is intended to be placed over the ulnar head (30).

The ulnar component (2) is manufactured in several sizes to make it possible to adjust the size of the prosthesis to the size of the ulnar head to be resurfaced.

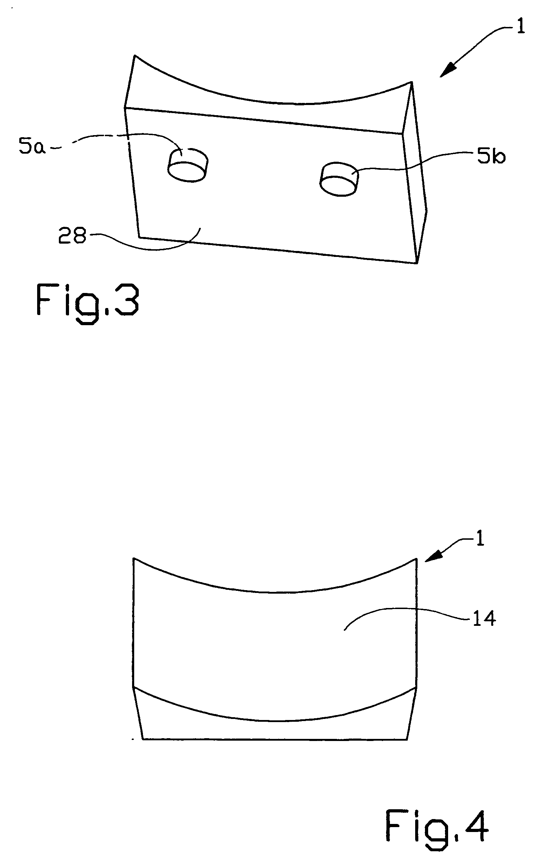

As shown in figures 3, 4, 7a and 7b, the radial component (1) has a concave (14) surface intended to face the ulnar component when inserted. Normally the concave surface is a part of a cylindrical surface but in some embodiments the surface is concave also in the longitudinal direction of the radial component as well. Usually the radial component (1), on the side (28) opposing the concave side (14), the radial component (1) is supplied with at least one protruding part (5a, 5b, 13), intended to be slid into a cut of the radius. The purpose of the protruding part is to secure the anchorage of the radial component to the radius. The protruding part could for example be formed as a keel (5a, 5b) or a shelf (13). The radial component 1, 1 a is made in different thicknesses so that by choosing the proper thickness, the joint and its ligaments can be adjusted to achieve optimal stability and range of motion.

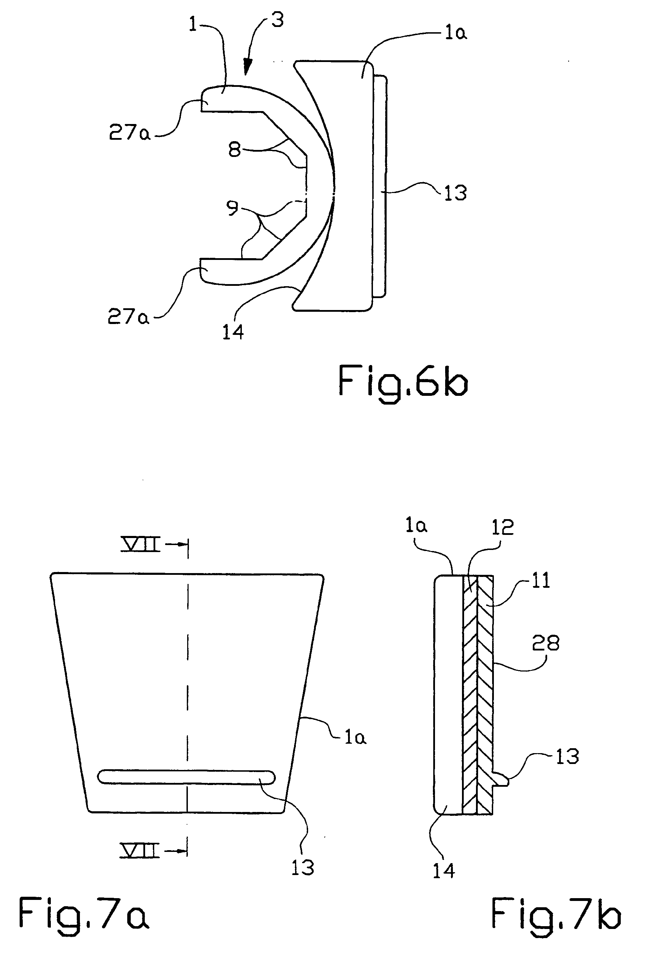

In the embodiment shown in figs. 7a and 7b, the radial component 1a comprises a bearing plate (12) and a base plate (11) The latter is normally made from metal.

The radial component (1, 1a) is manufactured in various lengths to make it possible at each and every operation to choose the right combination of length and thickness in relation to the size of the ulnar component (2) and the distance between the two cut surfaces of the radius and the ulnar head. The concave surface (14) of the radial component has a size sufficient to allow the ulnar component to be able to continuously abut against the concave surface of the radial component during pronation or supination to and from the two extreme positions of rotation.

The radial and the ulnar component (1, 1a) and (2, 2a) respectively, are usually anchored with or without bone cement (polymethylmetacrylat, PMMA) to the radius (6) and ulna (7) respectively.

The radial and ulnar components are designed so that the radius of the bending radius of external surface of the ulnar component (3.3a) is less than that of the concave surface of the radial component (14) in each part opposing each other during a full pivotation of the arm. By this design of the convex outer (3.3a) and concave (14) surfaces, the ulnar head is assured to move relative to the concave radial surface (14) in both a rolling as well as a translatory movement.

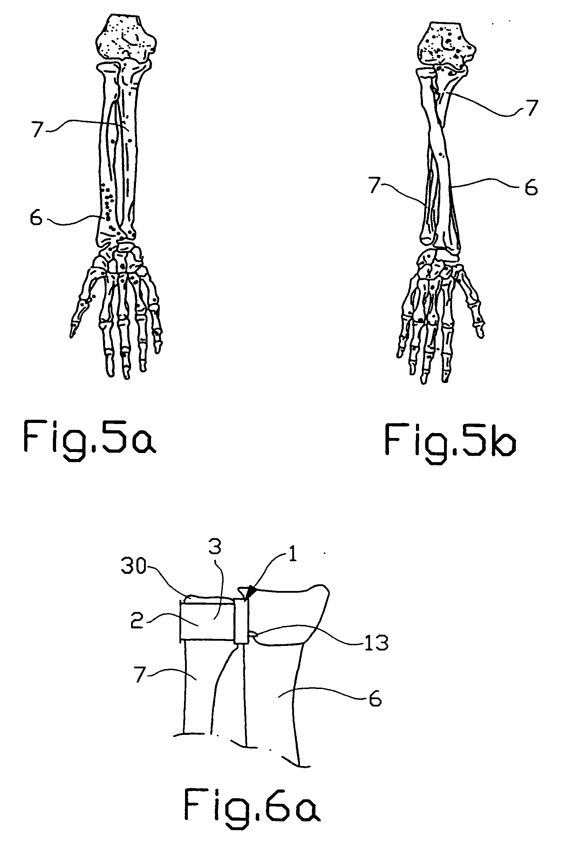

In fig. 5a, 5b is shown schematically how the radius (6) and the ulna (7) are moved relative to each other when the forearm is turned 180 degrees. In the figure one can see that the radius rolls around the ulna. According the invention, the radial component (1.1a) is designed to be placed in the distal part of the radius and the ulnar component (2.2a) to be placed on the ulnar head.

In figs. 6a and 6b, examples of the radial component (1.1a) and the ulnar component (2.2a) positions relative each other are shown in situ in the distal radioulnar joint. As seen in figs. 6a and 6b, the design of the radial (1.1a) and ulnar component (2.2a) allows the required rolling and translatory relative motion, as the arm is rotated, during which the ulna component, radially attached to the ulna head, moves along the external concave surface (14) of the radial component.

To prepare the radius (6) and ulna (7) for the surface replacement, the radial and ulnar bones have to be cut during the operation. The radial (6) cut leaves a flat surface and the ulnar cut leaves several mainly flat surfaces in order to receive the radial (1.1a) and ulnar component (2.2a). As previously noted the radius is prepared for the extruding part (13) or the parts 5a, 5b of the radial component. The protruding parts of the radial component (the keel or the shelfs) are intended to secure the anchoring of the radial component to the radial bone.

When the surface replacement according to the invention is implanted, the ulnocarpal ligaments and the triangular fibrocartilaginous complex (TFCC or the disc) normally are maintained. Thereby, the passive compressive forces of the distal radioulnar joint are maintained. A radial cut thus cannot be made where these ligaments insert to the bone i.e. distal to the fovea on the radius or on the styloid of the ulna. The radial (6) and ulnar (7) cuts for the surface replacements (1, 1a; 2, 2a) thus are made immediately proximal to the insertion of the radioulnar ligaments. On the radial cut a flat surface is formed with holes for the pegs (5a, 5b) or slots for the protruding shelf (13). On the ulna (7) rectangular angled cuts are prepared that will fit the contour (9) of the internal surface (2) of the ulnar component (4).

During the operation, the cut and prepared ulnar end (7) can be pivoted to facilitate the cut and the mounting of the ulnar component (2.2a). For the radius it is difficult to reach the whole cut bone surface and the radial component have to be slid down into place between the ulna and radius. In the designs with pegs (5), these are pushed into prepared holes or slots of the radius. Hereby, the radial and the ulnarar components are stabilized in their positions on the radius and ulna.

The orientation of the distal joint surfaces of the radius and ulna varies between individuals. In spite of this, a standard orientation of the joint surfaces can be used for the radial and ulnar component after being positioned on radius and ulna respectively, provided that the components fit. An adjustment is possible intraoperatively when the cuts are made. In the embodiment where the outer surface of the ulnar component (3a) is convex also in the longitudinal direction, the standard orientation normally will be accepted by the joint surface of the radial component. Since both components are immediately mechanically stabile, postoperative motion can be started immediately by the patient.

The description above demonstrates a limited number of possible designs of the invention. The professional realizes that the invention hosts a vast number of modifications and embodiments within the framework of the following patent claims.

## Claims

1. A surface replacement for the distal radioulnar joint, comprising a radial component (1) and an ulnar component (2), **characterized in that** it comprises:
surfaces (14, 3) in congruity with each other, and
each component is intended to be placed on radius (6) and ulna (7) respectively, so as to form a new surface at the natural joint surface, said new surface thereby permitting the ulnar component to move relative to the radial component in both a rolling and a translatory movement.

2. The replacement of claim 1, **characterized in that** the components (1, 2) after implantation are arranged to be facing each other during rotational motion of the forearm.

3. The replacement of claim 1 or 2, **characterized in that** the ulnar component (2) has a mainly convex outer surface and that the ulnar component (2) has an inner surface of quadratic or rectangular fields angled to each other.

4. The replacement of claim 3, **characterized in that** the ulnar component (2) comprises a number of juxtapositioned and integrated mainly quadratic or rectangular elements (27, 27a).

5. The replacement of claim 4, **characterized in that** out of said elements (27, 27a), the two outermost positioned ones (27a) are parallel to each other.

6. The replacement according to any one of the previous claims, **characterized in that** the radial component (1) has a concave surface (14) intended to, after implantation, be faced towards the ulnar component (2) and during pivotation in the distal radioulnar joint, bear on to the outer surface of the ulnar component (3).

7. The replacement according to claim 6 as depending from claim 3, wherein the radius of the bending radius of the external surface of the ulnar component (3, 3a) is less than that of the concave surface of the radial component (14).

8. The replacement according to any one of the previous claims, **characterized in that** the radial component (1) opposite the concave surface (14) has a surface (28) having at least one protruding part (5a, 5c, 13) intended to be inserted into holes or slots of the radius.

9. The replacement of claim 8, **characterized in that** the extruding part is formed as one or more pegs (5a, 5b), or as an elongate shelf (13).

10. The replacement of claims 6-9, **characterized in that** the radial (1) component comprises a base anchoring plate (11) onto which and an articulating plate (12) is fixed.

11. The replacement according to any one of the previous claims, **characterized in that** the radial and ulnar components (1) and (2) respectively, are capable of being anchored to the radius (6) and ulna (7) using bone cement.

12. An ulnar component (2) of a surface replacement for the distal radioulnar joint, **characterized by**:
a body (24) with a convex outer surface (3) forming a mainly semicylindrical envelope surface around a central axis (21), the convex surface serving to replace the natural joint surface of the ulna that can execute both a rolling and a translatory movement relative to the radial component of the radioulnar joint, and
an inner surface (4) having rectangular fields forming angles to each other that are to be pressed against the ulna by the passive compressive forces of the distal radioulnar joint.

13. A radial component (1) of a surface replacement for the distal radioulnar joint, **characterized by** a side (28) having at least one protruding part (5a, 5b, 13) to be pressed against the radius by the passive compressive forces of the distal radioulnar joint, said side opposing a concave side (14) which is a replacement for the natural joint surface of the radius that permits the ulnar component to move relative to the radial component in both a rolling and a translatory movement.

## Patentansprüche

1. Oberflächenersatz für das distale Radioulnargelenk, umfassend eine radiale Komponente (1) und eine ulnare Komponente (2), **dadurch gekennzeichnet, dass** er umfasst:
in Kongruenz miteinander stehende Oberflächen (14, 3) und
wobei jede Komponente dazu gedacht ist, auf die Speiche (6) bzw. die Elle (7) gelegt zu werden, um so eine neue Oberfläche an der natürlichen Gelenkoberfläche zu bilden, wobei es die neue Oberfläche **dadurch** der ulnaren Komponente erlaubt, sich relativ zur radialen Komponente sowohl in einer Roll- als auch in einer Translationsbewegung zu bewegen.

2. Ersatz nach Anspruch 1, **dadurch gekennzeichnet, dass** die Komponenten (1, 2) nach der Implantierung so angeordnet sind, dass sie während der Drehbewegung des Unterarms einander zugewandt sind.

3. Ersatz nach Anspruch 1 oder 2, **dadurch gekennzeichnet, dass** die ulnare Komponente (2) eine hauptsächlich konvexe äußere Oberfläche besitzt und dass die ulnare Komponente (2) eine innere Oberfläche aus zueinander abgewinkelten quadratischen oder rechteckigen Feldern besitzt.

4. Ersatz nach Anspruch 3, **dadurch gekennzeichnet, dass** die ulnare Komponente (2) eine Anzahl von nebeneinander liegenden und integrierten, hauptsächlich quadratischen oder rechteckigen Elementen (27, 27a) umfasst.

5. Ersatz nach Anspruch 4, **dadurch gekennzeichnet, dass** von den besagten Elementen (27, 27a) die beiden am weitesten außen positionierten (27a) parallel zueinander sind.

6. Ersatz nach einem der vorangehenden Ansprüche, **dadurch gekennzeichnet, dass** die radiale Komponente (1) eine konkave Oberfläche (14) aufweist, die dazu gedacht ist, nach der Implantation zur ulnaren Komponente (2) gewandt zu sein und während der Drehbewegung im distalen Radioulnargelenk auf der äußeren Oberfläche der ulnaren Komponente (3) aufzuliegen.

7. Ersatz nach Anspruch 6, wenn abhängig von Anspruch 3, wobei der Radius des Biegungsradius der äußeren Oberfläche der ulnaren Komponente (3, 3a) geringer ist als jener der konkaven Oberfläche der radialen Komponente (14).

8. Ersatz nach einem der vorangehenden Ansprüche, **dadurch gekennzeichnet, dass** die radiale Komponente (1) gegenüber der konkaven Oberfläche (14) eine Oberfläche (28) besitzt, die mindestens einen vorstehenden Teil (5a, 5c, 13) aufweist, der dazu gedacht ist, in Löcher oder Schlitze der Speiche eingesetzt zu werden.

9. Ersatz nach Anspruch 8, **dadurch gekennzeichnet, dass** der vorstehende Teil als ein oder mehrere Stifte (5a, 5b) oder als länglicher Absatz (13) ausgebildet ist.

10. Ersatz nach Ansprüchen 6 bis 9, **dadurch gekennzeichnet, dass** die radiale Komponente (1) eine Basisverankerungsplatte (11) umfasst, auf welcher eine Gelenkplatte (12) befestigt ist.

11. Ersatz nach einem der vorangehenden Ansprüche, **dadurch gekennzeichnet, dass** die radiale und ulnare Komponente (1) bzw. (2) in der Lage sind, unter Verwendung von Knochenzement an der Speiche (6) bzw. der Elle (7) verankert zu werden.

12. Ulnare Komponente (2) eines Oberflächenersatzes für das distale Radioulnargelenk, **gekennzeichnet durch**:
einen Körper (24) mit einer konvexen äußeren Oberfläche (3), die um eine mittlere Achse (21) eine hauptsächlich halbzylindrische Hüllfläche bildet, wobei die konvexe Oberfläche dazu dient, die natürliche Gelenkoberfläche der Elle zu ersetzen, welche sowohl eine Roll- als auch eine Translationsbewegung in Bezug auf die radiale Komponente des Radioulnargelenks ausführen kann, und
eine innere Oberfläche (4) mit Winkel zueinander bildenden rechteckigen Feldern, die **durch** die passiven Kompressionskräfte des distalen Radioulnargelenks auf die Elle zu pressen sind.

13. Radiale Komponente (1) eines Oberflächenersatzes für das distale Radioulnargelenk, **gekennzeichnet durch** eine Seite (28) mit mindestens einem vorstehenden Teil (5a, 5b, 13), der **durch** die passiven Kompressionskräfte des distalen Radioulnargelenks auf die Speiche zu pressen ist, wobei die Seite einer konkaven Seite (14) gegenüberliegt, welche ein Ersatz für die natürliche Gelenkoberfläche der Speiche ist, der es der ulnaren Komponente erlaubt, sich relativ zur radialen Komponente sowohl in einer Roll- als auch einer Translationsbewegung zu bewegen.

## Revendications

1. Remplacement de surface pour l'articulation radio-cubital distale comportant une pièce radiale (1) et une pièce cubitale (2), **caractérisé en qu'**il comprend :
des surfaces (14, 3) conformes entre elles, et
chaque pièce est prévue pour être placée sur le radius (6) et le cubitus (7), respectivement, afin de former une nouvelle surface à la surface de l'articulation naturelle, ladite nouvelle surface permettant ainsi à la pièce cubitale de se déplacer par rapport à la pièce radiale à la fois en un mouvement de roulement et en un mouvement de translation.

2. Remplacement selon la revendication 1, **caractérisé en ce que** les pièces (1, 2) après l'implantation sont agencées de façon à être face à face pendant un mouvement de rotation de l'avant-bras.

3. Remplacement selon la revendication 1 ou 2, **caractérisé en ce que** la pièce cubitale (2) a une surface extérieure principalement convexe et **en ce que** la pièce cubitale (2) a une surface intérieure de champs quadratiques ou rectangulaires inclinés entre eux.

4. Remplacement selon la revendication 3, **caractérisé en ce que** la pièce cubitale (2) comporte un certain nombre d'éléments (27, 27a) principalement quadratiques ou rectangulaires, juxtaposés et intégrés.

5. Remplacement selon la revendication 4, **caractérisé en ce que** parmi lesdits éléments (27, 27a), les deux éléments (27a) positionnés le plus à l'extérieur sont parallèles entre eux.

6. Remplacement selon l'une quelconque des revendications précédentes, **caractérisé en ce que** la pièce radiale (1) a une surface concave (14) prévue pour être placée, après l'implantation, face à la pièce cubitale (2) et pour porter sur la surface extérieure de la pièce cubitale (3) pendant un pivotement dans l'articulation radio-cubitale distale.

7. Remplacement selon la revendication 6, lorsqu'elle dépend de la revendication 3, dans lequel le rayon du rayon de courbure de la surface extérieure de la pièce cubitale (3, 3a) est inférieur à celui de la surface concave de la pièce radiale (1).

8. Remplacement selon l'une quelconque des revendications précédentes, **caractérisé en ce que** la pièce radiale (1), opposée à la surface concave (14), a une surface (28) ayant au moins une partie saillante (5a, 5c, 13) prévue pour être insérée dans des trous ou des rainures du radius.

9. Remplacement selon la revendication 8, **caractérisé en ce que** la partie saillante est réalisée sous la forme d'un ou plusieurs tenons (5a, 5b) ou d'un plateau allongé (13).

10. Remplacement selon les revendications 6 à 9, **caractérisé en ce que** la pièce radiale (1) comporte une plaque d'ancrage de base (11) sur laquelle est fixée une plaque d'articulation (12).

11. Remplacement selon l'une quelconque des revendications précédentes, **caractérisé en ce que** les pièces radiale et cubitale (1) et (2), respectivement, peuvent être ancrées au radius (6) et au cubitus (7) en utilisant un ciment osseux.

12. Pièce cubitale (2) d'un remplacement de surface pour l'articulation radio-cubitale distale, **caractérisée par** :
un corps (24) ayant une surface extérieure convexe (3) formant une surface d'enveloppe principalement semi-cylindrique autour d'un axe central (21), la surface convexe servant à remplacer la surface d'articulation naturelle du cubitus qui peut exécuter à la fois un mouvement de roulement et un mouvement de translation par rapport à la pièce radiale de l'articulation radio-cubitale, et
une surface intérieure (4) ayant des champs rectangulaires formant des angles entre eux afin d'être appliqués sous pression contre le cubitus par les forces passives de compression de l'articulation radio-cubitale distale.

13. Pièce radiale (1) d'un remplacement de surface pour l'articulation radio-cubitale distale, **caractérisée par** un côté (28) ayant au moins une partie saillante (5a, 5b, 13) destinée à être appliquée sous pression contre le radius par les forces de compression passives de l'articulation radio-cubitale distale, ledit côté étant opposé à un côté concave (14) qui est un remplacement pour la surface d'articulation naturelle du radius qui permet à la pièce cubitale de se déplacer par rapport à la pièce radiale à la fois en un mouvement de roulement et un mouvement de translation.
